# EUROPEAN PATENT SPECIFICATION

(11) **EP 4 122 388 B1**
(45) Date of publication and mention of the grant of the patent: **22.05.2024**
(21) Application number: 22185935.8
(22) Date of filing: 20.07.2022
(51) Int. Cl.: A61B 5/08, A61B 5/097, G01N 33/497

(54) **A METHOD FOR COLLECTING BREATH SAMPLES**
VERFAHREN ZUM SAMMELN VON ATEMPROBEN
PROCÉDÉ DE COLLECTE D'ÉCHANTILLONS D'EXPIRATION

(30) Priority: 20.07.2021 PL 43853421
(43) Date of publication of application: 25.01.2023
(73) Proprietor: Gdanski Uniwersytet Medyczny, 80-210 Gdansk (PL); Politechnika Gdanska, 80-233 Gdansk (PL)
(72) Inventor: B czek, Tomasz, 80-174 Gda sk (PL); Konieczna, Lucyna, 84-200 Reda (PL); Siebert, Janusz, 80-299 Gda sk (PL); Krawczuk, Marek, 80-283 Gda sk (PL); Doli ski, ukasz, 80-169 Gda sk (PL); Redlarski, Grzegorz, 80-297 Banino (PL)
(74) Representative: JWP Patent & Trademark Attorneys

(56) References cited:
- WO-A1-2021/089992
- GB-A- 2 548 122
- US-A1- 2010 268 106
- US-A1- 2020 033 323

## Description

### Field of invention

The object of the invention is a method for collecting breath samples.

### Prior art

Disclosed in the prior art are solutions concerning the collection of excretion in the form of water vapour condensate contained in the air exhaled by the patient.

Disclosed in international patent application no. WO 2019/018121 is a device for the collection and storage of samples in the form of exhaled breath condensates. The device, however, features no elements which would cool down the collected breath samples.

Disclosed in the description of invention IT 102011901967915 A is a device for the collection of air samples which incorporates a condenser where the sample gets cooled down. The device, however, is expensive in use and features a different method of controlling the temperature of the cooling block.

Disclosed in the description of invention GB2548122 A is a kit of parts for an exhaled breath condensate collection device, which comprises a mouthpiece module comprising a breath passageway defined in the mouthpiece module providing fluid conduction from a mouthpiece breath inlet port for receiving exhaled breath to a mouthpiece breath outlet port in use. The kit further comprises a collection vessel for insertion into the device for cooling in use. The collection vessel defines a sealed and resealable chamber for collecting exhaled breath condensate in use. The collection vessel has a vessel breath inlet for admitting exhaled breath into the chamber. The kit of parts is configured such that the collection vessel is: insertable into the device into an sample collection configuration in which the vessel breath inlet is unsealed and in fluid communication with the mouthpiece breath outlet port of the mouthpiece module; and removable from the device in a sample containment configuration in which the collection vessel chamber is resealed. The device and method do not discloses cooling block with an active element and a system for regulation and controlling the cooling process of the condensate container.

Disclosed in the description of invention US2020/033323 A1 is an analyser for collecting and analysing a breath condensate comprising a housing having a cooling means and, further, retaining a cartridge device comprising a condensation zone to condense exhaled breath from a subject, and one or more further discrete regions for detection of analyte and measurement of analyte, the cartridge device further comprising a fluid path connecting the condensation zone to the or each discrete region. The device, however, features no elements for the collection of breath samples comprising a mouthpiece, a saliva collector and sample receiving unit with active, integrated cooling system which ensure proper quality parameters of the collected air condensate sample.

Disclosed in the description of invention WO2021/089992 A1 is a exhaled breath collection device suitable for collection and measurement of a biomarker in exhaled breath. More particularly, the device comprising a sensor unit configured to measure a biomarker in exhaled breath, a cooling device configured to reduce a temperature of exhaled breath, and a temperature control unit, wherein the temperature control unit can control the cooling device to reach a target temperature greater than 0 °C and less than or equal to 30 °C, wherein the biomarker is hydrogen peroxide (H₂O₂).

### Detailed Description of the Invention

All known solutions concerning the collection of excretion employ the phenomenon of condensation of water vapour contained in the exhaled air. To that end, one should cool down the air below the dew point. The main element which differentiates individual existing solutions is the cooling method and the way of regulating temperature in the chamber where the condensate accumulates. The devices which condense water vapour contained in the air may be divided into constructions featuring an integrated or external system of discharging heat from the cooling chamber (active systems) and those in which the cooling block is preliminarily frozen, following which the heat is no longer discharged from the chamber during the condensation process (passive cooling).

Effectiveness of the condensate collection process depends directly on the device's ability to keep low temperature stable. When the patient exhales air during the test the cooling chamber heats up; therefore, it is necessary to look for solutions which would guarantee the possibility to keep the low temperature of the exhaled breath condensate sample at a stable level.

In the course of the conducted research, the Inventors solved the problem of obtaining a sample of exhaled breath condensate of proper quality by providing an apparatus serving the purpose. Moreover, it turned out that a sample of the exhaled breath condensate collected with the apparatus according to the invention enables a qualitative and quantitative analyses of the biological substances contained in the sample, which is useful when diagnosing aetiology of infections.

An object of the disclosure is to provide an apparatus for the collection of breath samples comprising a mouthpiece, a saliva collector and a sample receiving unit that consists of a unit which supplies the inhaled air, comprising the mouthpiece 1 connected to the saliva collector 2 at the bottom, and featuring a pipe 3 at its end, where the pipe 3 is detachably connected to the sample receiving unit via an inlet duct 4, wherein the sample receiving unit consists of a test tube 6 for exhaled breath condensate detachably connected at the bottom to a cooling block 5 characterised in that the cooling block 5 is fitted with a cooling chamber 5a, an active element of the cooling block 5b, a temperature sensor 5c connected to a heat dispersion unit 5d via a system of heat pipes, and where the active element of the cooling block 5b is controlled via a programmable control unit 5e featuring an LCD display and a keyboard which enables setting and monitoring of the apparatus working parameters, and powered by an electric unit 5f, and wherein the cooling block 5 is detachably connected to an outlet duct 7 to which the unit which forces one-direction flow 8 is connected, wherein the outlet duct 7 and unit 8 are in a parallel position with respect to the inlet duct 4.

The apparatus features an active, integrated cooling system which ensures proper quality parameters of the collected air condensate sample.

An important element which differs the solution from the known devices is the method of controlling the temperature of the cooling block **5.** Usually, it is achieved by way of the double-state method which consists in defining the fixed lower (T1) and upper (T2) threshold of the temperature in the cooling chamber **5a.** When the cooling chamber reaches the pre-set temperature T1, the cell switches off and remains inactive until the threshold temperature T2 is reached. When it occurs, the cell switches on and continues to work until temperature T1 is reached again. In practice, however, the temperature in the chamber continues to increase above threshold temperature T2 despite the work of the cooling system because of the thermal inertia of the system. This can cause substantial temperature changes which impair effectiveness of the excretion condensation process and the quality of the condensate.

The object of the invention is defined by a method of collecting a breath samples, which involves exhaling air from the lungs and collecting the samples, wherein the air exhaled from the lungs through the mouthpiece **1** is directed via the pipe **3** to the sample receiving unit, where the air supplied through the inlet duct **4** cools down in the cooling block **5** to a temperature below the dew point, in effect of which the water vapour contained in the exhaled air condenses and accumulates in the test tube **6.**

The active element of the cooling block **5** used in the method according to the invention is the Peltier cell **5b.** In the method according to the invention the cooling chamber **5a** is first cooled down to temperature T1 pre-set at -10 °C, whereupon the power supplied to the Peltier cell **5b** is reduced so as to keep the temperature of the cooling chamber **5a** at the pre-set level, following which the cooling block **5** is connected to the unit which supplies the inhaled air and to the sample receiving unit, following which the patient exhales air from the lungs (for approximately 20 minutes), and once the air is supplied to the cooling block **5** it gets cooled down below the dew point in effect of which the water vapour contained in the exhaled air condensates and the excretion intended for further testing in the form of exhaled breath condensate accumulates on the walls of the test tube **6,** and where the unit forcing one-direction flow **8** enables the discharging of the gases and keeping normal pressure inside the test tube **6.** When the condensate sample is being collected, the cooling chamber **5a** heats up in contact with the warm air exhaled from the patient's lungs; that is why the programmable control unit **5e** controls the power supplied to the cell **5f** so as to keep the pre-set temperature at a stable level within a narrow temperature fluctuation margin range, which enables keeping thermal balance in the cooling chamber **5a.**

Once an appropriate volume of the exhaled breath condensate has been collected, the test tube **6** is disconnected and the accumulated condensate is used in analyses or subject to further processing aimed at fixing the sample, such as freezing or lyophilisation.

Another object not forming part of the invention is a method of diagnosing medical condition of the patient characterised in that the breath sample collected as described above is subject to a quantitative or qualitative analysis.

The inventors of the invention conducted an analysis of the exhaled breath condensates collected using the apparatus according to the invention among paediatric patients suffering from acute lymphoblastic leukemia (ALL), children with diabetes type 1, and bariatric patients. Observed were statistically significant differences in the amino acid profiles measured in the exhaled air (before and after the treatment).

The results yielded the analyses of the amino acid profiles of the samples of exhaled breath condensates collected from patients treated for the diagnosed acute respiratory distress syndrome (ARDS) proved particularly interesting. It was demonstrated that disease aetiology can be differentiated based on an analyses of differences in the levels of amino acids, especially in the case of viral infections, including those caused by SARS-COV 2. Hence, the solution according to the invention can be potentially used in diagnosing diseases of viral origin, such as the acute respiratory distress syndrome (ARDS) or COVID-19.

The technological readiness of the solutions according to the invention in the TRL scale has been assessed at level VII.

### Description of the Figures

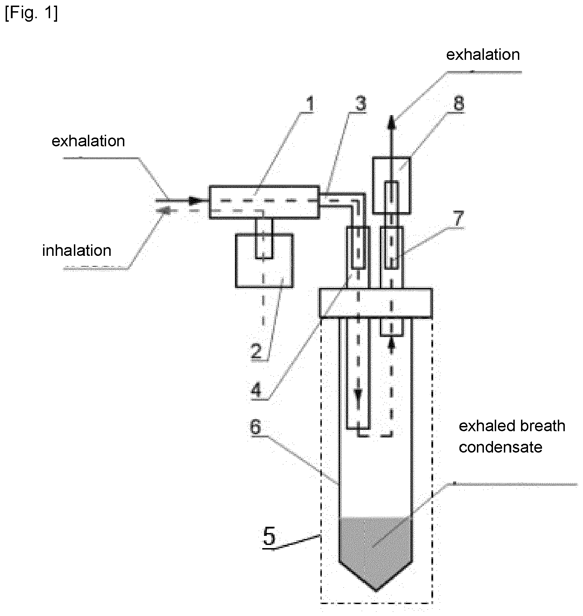

The subject matter of the invention is shown on drawing figures, where
[Fig. 1] presents the schematic diagram of the structure of the apparatus for collecting samples of the exhaled air. The arrows indicate air flow direction;

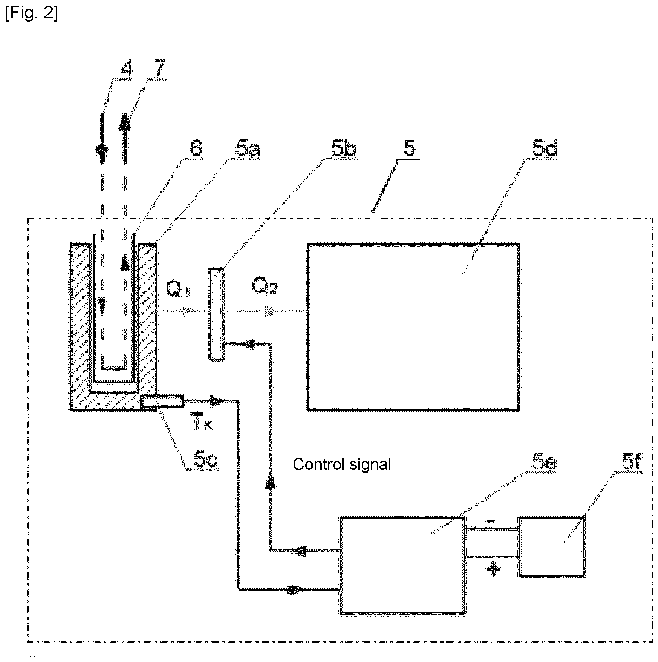

[Fig.2] presents the cooling block **5**
[Fig.3] presents the diagram of the amino acid derivatisation procedure
[Fig.4] presents a photograph of a prototype cooling block according to the invention

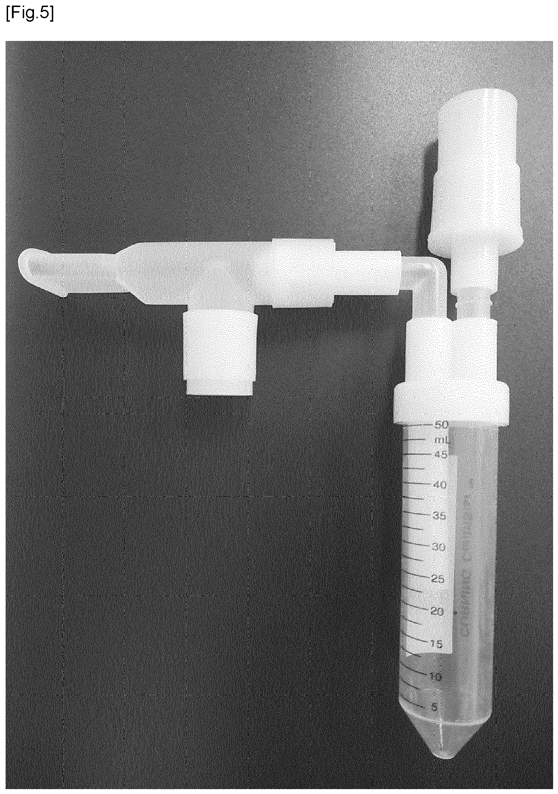

[Fig.5] presents a photograph of the unit which supplies the inhaled air and the receiving unit

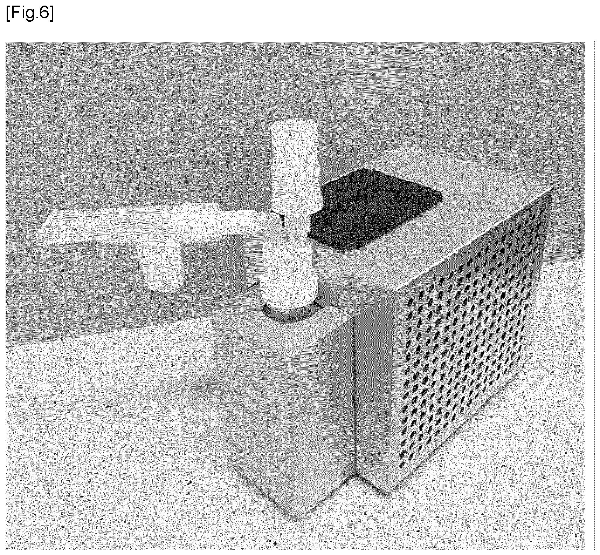

[Fig.6] presents a photograph of a prototype apparatus for collecting samples of exhaled breath condensates

List of numerical references:
1 - mouthpiece
2 - saliva collector
3 - pipe
4 - inlet duct
5 - cooling block
5a - cooling chamber
5b - Peltier cell
5c - temperature sensor
5d - heat dispersion unit
5e - programmable control unit with a display and keyboard
5f - power supply unit
6- test tube
7- discharge duct
8- unit forcing one-direction flow

### Experimental part

The invention is highlighted in the following embodiments, which do not limit its scope.

### Example 1

The apparatus according to the invention which was used incorporated the Peltier cell **5b** as an active element of the cooling block **5.**

The cooling chamber **5a** was cooled down to temperature T1 pre-sent at -10 °C, whereupon power supply to the Peltier cell **5b** was reduced so as to keep the temperature of the cooling block **5** at the pre-set level, following which the cooling block **5** was connected to the unit which supplies the inhaled air and to the sample receiving unit. The patient was exhaling air from the lungs to the mouthpiece for 20 minutes, the air, when supplied to the cooling block **5,** was cooled down to -7 °C, in effect of which the water vapour contained in the exhaled air condensed, and the excretion in the form of exhaled breath condensate intended for further testing started to accumulate on the walls of the test tube **6,** while the exhaled gases were removed from the test tube **6** by the unit forcing one-direction flow **8.** The control unit **5e** had been set up to keep the pre-set temperature at a stable level, with minor temperature fluctuations remaining within the range of ±3°C which enabled keeping the thermal balance in the cooling block **5.**

### Example 2

### Exhaled breath condensates (EBC)

EBC samples are a valuable diagnostic material collected from patients in a totally non-invasive and safe way. Samples of exhaled air are collected when the patient is breathing regularly and calmly and their collection is not inconvenient or tiresome to him/her [1].

The tested EBC samples were collected from healthy volunteers participating in the research project conducted in cooperation with the MUG's Immunology Clinic, on approval no. 274/2018 from the Bioethical Commission. All participants taking part in the research gave their written consent and were asked to complete a brief questionnaire.

EBC samples were collected on the same day, on an outpatient basis, at the Clinic of General, Endocrine, and Transplant Surgery at the MUG's Invasive Medicine Centre (CMI). Collection of the samples was performed under the supervision of clinicians. Breath samples were collected by qualified medical staff during an appointment with a physician. On the same day the collected material was transported to the MUG's Chair and Department of Pharmaceutical Chemistry and stored deeply frozen in the temperature of -80°C until the time a LC-MS/MS analysis was performed.

The EBC samples were collected with the apparatus according to the invention (as in Example 1) and the commercially available device called Medivac Turbo DECCS, following the manufacturer's user manual.

The EBC samples were subject to the quantitative analysis of the amino acids present in the condensate.

### The amino acid derivatisation procedure

Amino acids are small molecule compounds difficult to assay in a reversed phase (RP) system. Because of their hydrophilic nature the molecules do not dissociate in such conditions. To improve detection, amino acids were subject to the derivatisation procedure which increased the mass of the monitored ions. In effect, their identification by the mass detector improved, which translated to the higher sensitivity of the method and enhanced analyte signal at the expense of reduced signals generated by noise. This resulted in achieving a low LOD value at the fmol level) [2]. In addition, the properties of amino acids changed from hydrophilic to lipophilic, which made it possible to use the C18 column and enabled perfect separation of the assayed analytes in the reversed phase system. The derivatisation was carried out in the pre-column mode, using propyl chloroformate as the derivatizing agent. Because of the short derivatization time and mild conditions (1 min, room temperature), the agent stands out among other earlier used reagents mentioned in the literature.

To that end, samples were transferred to glass 1.2 mL test tubes using an automatic pipette so as to subject them to the process of derivatization.

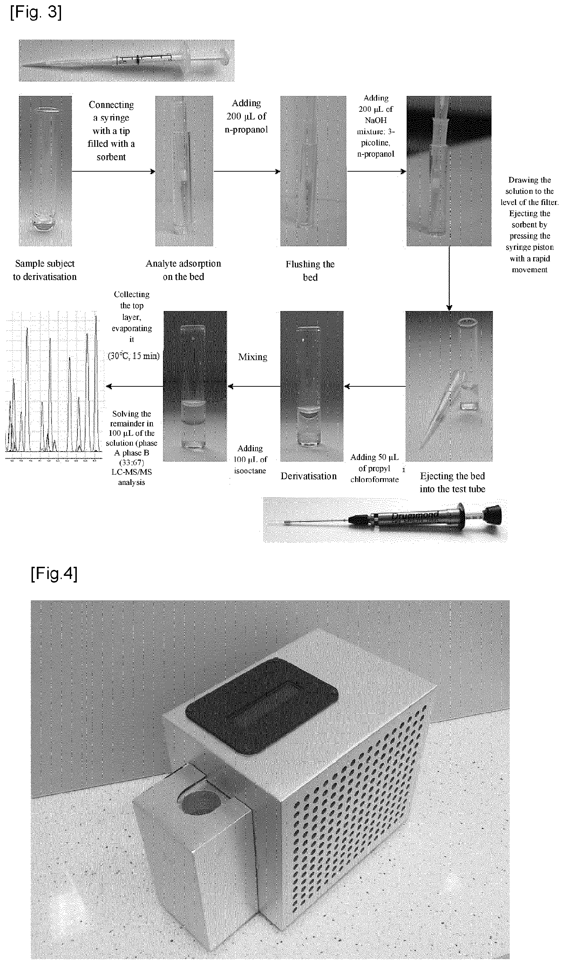

A solution of NaOH and 3-picoline in n-propanol was prepared in the volumetric proportion of 3:2 (v/v), 200 µL per a test tube. Pipette ends filled with a bed were connected to a syringe which was used to place the solution inside the test tube on the sorbent where the analytes got adsorbed. After completion of this phase, the bed was washed with 200 µL of n-propanol. The solution prepared earlier was used to wash out the bed from the pipette end to the glass test tube. 50 µL of the derivatizing agent (propyl chloroformate in chloroform) was added, following which the mixing with a shaker was carried out for about 8 seconds until white emulsion was obtained. The mixing was repeated after one minute. Then 100 µL of isooctane was added and the mixing was performed again. The top organic layer (approximately 150 µL) was collected with a pipette and subject to evaporation in vacuum at the temperature of 30°C for 15 min. The dry remains were solved in 100 µL of a mobile phase mixture (phase A: 10mM of ammonium formate: phase B: 10 mM of ammonium formate in methanol (33:67, v/v) and subject to the LC-MS/MS analysis. A diagram of the amino acid derivatization procedure is shown in Fig.3.

The advantage of the amino acid derivatizing agent in the form of propyl chloroformate used in the invention comes down to its easy availability in the market and low price. Moreover, the amino acid derivatisation procedure is swift, and it is possible to derivatise amino acids in a water solution directly. Then, the derivatised compounds are extracted using an organic solvent.
The conditions of assaying amino acids in exhaled breath condensate (EBC) using the LC-MS/MS technique.

The derivatized amino acids were assayed using the technique of liquid chromatography coupled with tandem mass spectrometry (LC-MS/MS). The mass spectrometer was fitted with a ion source of the electrospray (ESI) type and an analyser of the triple quadrupole type (MS/MS). Amino acids were separated on the C18 column (Phenomenex EZ: faast AAA-MS) sized 25 x 2.0 mm; 4 µm in the reversed phase (RP) system. Gradient elution was used (Tab. 2). The mobile phase A was made up of water with 10 mM of ammonium formate and 0.1 % formic acid added to it. Phase B consisted of methanol with 10 mM of ammonium formate and 0.1 % formic acid added to it.

### Conditions of the gradient elution

| Conditions of the gradient elution | |
|---|---|
| Time, min | % of phase B |
| 0 →13.5 | 63 →85 |
| 13.5 → 13.6 | 85 →63 |
| 13.6 → 18 | 63 |
| Flow: | 0.3 mL/min |

The optimised conditions of analytical separation of amino acids using the LC-MS/MS technique were as follows:
- injection volume: 1 µL
- total time of a single analysis: 18 min
- flow of the mobile phase: 0.3 mL/min
- column temperature: 35°C
- autosampler temperature: 4°C
- MS mode: positive
- temperature of the source of ESI ions: 300°C
- temperature of the heating block: 400°C
- flow of the heating gas (nitrogen): 10 L/min
- flow of the drying gas (nitrogen): 10 L/min
- flow of the nebulizing gas: 3 L/min
- temperature of the desolvation line (DL): 250°C
- capillary voltage: 5 kV

The results of the measured amino acid concentration are given in Table 1. Table 1 presents the results of measurements of the level of amino acids [ng/mL] assayed by a proprietary method employing the LC-MS/MS technique. The meanings of the acronyms: Turbo DECCS means an apparatus for the collection of exhaled breath condensate, manufactured by an Italian company and available in the market on commercial terms; PG apparatus means the apparatus according to the invention; NO denotes a non-detectable concentration (value below the detection threshold of the developed analytical method).

**Table 1**

| | | | Concentration [ng/mL] | | | | |
|---|---|---|---|---|---|---|---|
| | | Standard concentrat ion 50 ng/mL | Person 1 | Person 2 | Person 3 | Person 4 | Person 5 |
| 1 | ARGININE | 49.894 | 11.412 | 3.712 | 3.256 | 3.398 | 15.904 |
| 2 | HOMOARGININE | 48.915 | NO | NO | NO | NO | NO |
| 3 | GLUTAMINE | 49.924 | 0.404 | NO | NO | 0.733 | 7.831 |
| 4 | SERINE | 48.543 | 21.564 | 12.242 | 10.954 | 2.031 | 125.313 |
| 5 | ASPARAGINE | 49.212 | NO | NO | NO | NO | 2.458 |
| 6 | HYDROXYPROLINE | 49.325 | NO | NO | NO | NO | 0.135 |
| 7 | GLICYNE | 49.356 | 10.912 | 3.712 | 4.012 | 2.145 | 10.563 |
| 8 | TREONINE | 48.632 | NO | NO | NO | NO | 9.956 |
| 9 | ALANINE | 49.478 | 81.534 | 40.506 | 42.592 | 17.721 | 110.584 |
| 10 | PROLINE | 49.525 | 12.842 | 5.298 | 6.032 | 7.855 | 59.204 |
| 11 | METHIONINE | 48.996 | NO | NO | NO | 0.204 | 1.255 |
| 12 | ASPARAGINE ACID | 49.658 | NO | NO | NO | NO | 13.204 |
| 13 | HISTIDINE | 49.323 | 9.036 | 2.104 | 3.112 | 4.108 | 44.533 |
| 14 | LYSINE | 49.912 | 1.098 | NO | 0.102 | 0.493 | 6.724 |
| 15 | NORVALINE | 49.864 | 28.212 | 15.012 | 15.956 | 13.134 | 49.996 |
| 16 | VALINE | 50.034 | 29.298 | 16.986 | 17.022 | 10.554 | 47.512 |
| 17 | GLUTAMIC ACID | 50.314 | 1.427 | 0.542 | 0.824 | 1.235 | 7.974 |
| 18 | TRYPTOPHAN | 50.854 | NO | NO | NO | NO | 2.808 |
| 19 | LEUCINE | 49.345 | 8.056 | 4.036 | 4.559 | 5.347 | 23.755 |
| 20 | PHENYLOALANINE | 49.722 | NO | NO | NO | NO | 7.145 |
| 21 | ISOLEUCINE | 48.956 | NO | 2.156 | 3.535 | 4.103 | 23.342 |
| 22 | CYSTEINE | 48.412 | 0.721 | 0.496 | 0.553 | 0.633 | 1.511 |
| 23 | TYROSINE | 50.936 | 0.808 | 1.512 | 3.354 | 4.574 | 19.936 |
| 24 | CYSTINE | 49.204 | 1.511 | 2.624 | 0.856 | NO | 2.845 |

### Example 3

### Exhaled breath condensates (EBC) - comparative research

EBC samples were collected and prepared as described in Example 2. The samples collected with the device according to the invention (PG apparatus) and a commercially available apparatus Turbo DESC were analysed for the concentration of the selected amino acid.

The results of the measured amino acid concentration are presented in Table 2.

**Table 2**

| | | Standard concentration 50 ng/mL | | **Person 1** | | **Person 2** | |
|---|---|---|---|---|---|---|---|
| | | Turbo DECC S | PG device | Turbo DECCS | PG device | Turbo DECCS | PG device |
| 1 | ARGININE | 49.064 | 49.894 | 10.064 | 11.412 | 3.46 | 3.712 |
| 4 | SERINE | 48.113 | 48.543 | 21.118 | 21.564 | 10.568 | 12.242 |
| 10 | PROLINE | 49.745 | 49.525 | 12.744 | 12.842 | 5.713 | 5.298 |
| 13 | HISTIDINE | 48.943 | 49.323 | 8.948 | 9.036 | 2.282 | 2.104 |
| 16 | VALINE | 49.755 | 50.034 | 28.755 | 29.298 | 15.413 | 16.986 |
| 17 | GLUTAMIC ACID | 50.225 | 50.314 | 1.223 | 1.427 | 0.358 | 0.542 |
| 24 | CYSTINE | 48.791 | 49.204 | 1.491 | 1.511 | 2.485 | 2.624 |

| | | **Person** 3 | | **Person 4** | | **Person** 5 | |
|---|---|---|---|---|---|---|---|
| | | Turbo DECCS | PG device | Turbo DECCS | PG device | Turbo DECCS | PG device |
| 1 | ARGININE | 2.944 | 3.256 | 3.204 | 3,398 | 14.032 | 15.904 |
| 4 | SERINE | 9.356 | 10.954 | 1.651 | 2,031 | 123.319 | 125.313 |
| 10 | PROLINE | 6.492 | 6.032 | 8.025 | 7.855 | 56.096 | 59.204 |
| 13 | HISTIDINE | 3.446 | 3.112 | 4.358 | 4.108 | 42.049 | 44.533 |
| 16 | VALINE | 15.758 | 17.022 | 9.498 | 10.554 | 45.36 | 47.512 |
| 17 | GLUTAMIC ACID | 0.623 | 0.824 | 0.985 | 1.235 | 7.116 | 7.974 |
| 24 | CYSTINE | 0.746 | 0.856 | NO | NO | 2.735 | 2.845 |

Conclusions: the samples collected with the apparatus according to the invention (PG apparatus) yielded more accurate results of amino acid concentrations which proves that collecting samples with a PG apparatus in stable temperature conditions is important for the quality of analysis and further diagnostics of the patient's condition.

### Citation List

### Non Patent Literature

[1] I. Horváth, J. Hunt, P.J. Barnes, Exhaled breath condensate: methodological recommendations and unresolved questions, Eur. Resp. J., 26 (2005) 523-548.
[2] Y. Sakaguchi, T. Kinumi, T. Yamazaki, A. Takatsu, A novel amino acid analysis method using derivatization of multiple functional groups followed by liquid chromatography/tandem mass spectrometry, Analyst 140 (2015) 1965-1973.

## Claims

1. A method for collecting breath samples, which involves exhaling air from the lungs and collecting the samples using an the- apparatus comprising a mouthpiece, a saliva collector and a sample receiving unit that consists of a unit which supplies the inhaled air, comprising the mouthpiece (1) connected to the saliva collector (2) at the bottom and featuring a pipe (3) at its end, where the pipe (3) is detachably connected to the sample receiving unit via an inlet duct (4), wherein the sample receiving unit consists of a test tube (6) for exhaled breath condensate detachably connected at the bottom to a cooling block (5) fitted with a cooling chamber (5a) featuring a temperature sensor (5c) and an active element of the cooling block (5b) connected to a heat dispersion unit (5d) via a system of heat pipes, wherein the cooling block (5) is powered by an electric unit (5f) and controlled via a programmable control unit (5e) featuring an LCD display and a keyboard, wherein the cooling block (5) is detachably connected to an outlet duct (7) to which the unit which forces one-direction flow (8) is connected, **characterised in that** prior to collecting the sample, the cooling block (5) is cooled down to the temperature T1 pre-set at -10 °C, following which the cooling block (5) is connected via pipe (3) to the unit which supplies the inhaled air and via the outlet duct (7) to the unit which forces on-direction flow (8), following which the air exhaled from the lungs through the mouthpiece (1) is directed through the pipe (3) to the sample receiving unit in which the air supplied through the inlet duct (4) cools down in the cooling block (5) to a temperature below the dew point, in effect of which the water vapour contained in the exhaled air condenses and accumulates in the test tube (6), while the unit which force the one-direction flow (8) is used to discharge the gases, and once an appropriate amount of exhaled breath condensate is collected, the test tube (6) is disconnected, wherein the breath sample from the test tube (6) in the form of water vapour condensate is used in analyses or subject to further processing aimed at fixing the sample, such as freezing or lyophilisation and subject to quantitative and/or qualitative analysis which includes analysis of the amino acid profile.

2. The method according to Claim 1, wherein the Peltier cell is used as the active element of the cooling block (5b), with the power supplied of the Peltier cell (5b) is set so that it is increased in order to cool the cooling block (5) down, and in order to keep the temperature of the cooling block (5) at the pre-set level it is reduced, wherein the power supplied of the Peltier cell (5b) is controlled with the control unit (5e).

## Patentansprüche

1. Ein Verfahren zum Sammeln von Atemproben, das das Ausatmen von Luft aus den Lungen und das Sammeln der Proben unter Verwendung einer Vorrichtung umfasst, die ein Mundstück, einen Speichelsammler und eine Probenaufnahmeeinheit umfasst, die aus einer Einheit besteht, die die eingeatmete Luft zuführt, umfassend das Mundstück (1), das unten mit dem Speichelsammler (2) verbunden ist und an seinem Ende ein Rohr (3) aufweist, wobei das Rohr (3) über einen Einlasskanal (4) lösbar mit der Probenaufnahmeeinheit verbunden ist, wobei die Probenaufnahmeeinheit aus einem Reagenzglas (4) besteht, 6) für ausgeatmetes Atemkondensat, das unten lösbar mit einem Kühlblock (5) verbunden ist, der mit einer Kühlkammer (5a) ausgestattet ist, die einen Temperatursensor (5c) und ein aktives Element des Kühlblocks (5b) aufweist, das über ein System von Wärmerohren mit einer Wärmeabgabeeinheit (5d) verbunden ist, wobei der Kühlblock (5) von einer elektrischen Einheit (5f) angetrieben und über eine programmierbare Steuereinheit (5e) mit einem LCD-Display und einer Tastatur gesteuert wird, wobei der Kühlblock (5) lösbar mit einem Auslasskanal (7) verbunden ist, an den die Einheit angeschlossen ist, die den einseitig abfließenden Strom (8) erzwingt, **dadurch gekennzeichnet, dass** der Kühlblock (5) vor dem Sammeln der Probe auf die bei -10 °C voreingestellte Temperatur T1 abgekühlt wird, wonach der Kühlblock (5) über eine Leitung (3) mit der Einheit verbunden ist, die die eingeatmete Luft zuführt, und über den Auslasskanal (7) mit der Einheit, die den einseitig abfließenden Strom (8) erzwingt, wonach die Luft aus den Lungen durch das Mundstück (1) wird durch die Leitung (3) zur Probenaufnahmeeinheit geleitet, in der sich die durch einen Einlasskanal (4) zugeführte Luft im Kühlblock (5) auf eine Temperatur unter dem Taupunkt abkühlt, wodurch der in der ausgeatmeten Luft enthaltene Wasserdampf kondensiert und sich im Reagenzglas (6) ansammelt, während die Einheit, die den einseitig abfließenden Strom (8) erzwingt, zum Abführen der Gase verwendet wird, und sobald eine entsprechende Menge an ausgeatmetem Atemkondensat gesammelt ist, wird das Reagenzglas (6) getrennt, wobei die Atemprobe aus dem Reagenzglas (6) in Form von Wasserdampfkondensat in Analysen verwendet wird oder einer weiteren Verarbeitung unterzogen wird, die darauf abzielt, die Probe zu fixieren, wie zum Beispiel Einfrieren oder Gefriertrocknen, und einer quantitativen und/oder qualitativen Analyse unterzogen wird, die eine Analyse des Aminosäureprofils beinhaltet.

2. Das Verfahren nach Anspruch 1, wobei die Peltier-Zelle als aktives Element des Kühlblocks (5b) verwendet wird, wobei die zugeführte Leistung der Peltier-Zelle (5b) so eingestellt wird, dass sie erhöht wird, um den Kühlblock (5) abzukühlen, und um die Temperatur des Kühlblocks (5) auf dem voreingestellten Niveau zu halten, wird sie reduziert, wobei die zugeführte Leistung der Peltier-Zelle (5b) mit der Steuereinheit (5e) gesteuert wird.

## Revendications

1. Procédé pour collecter des échantillons d'haleine, qui consiste à expirer l'air des poumons et collecter les échantillons en utilisant un appareil comprenant un embout, un collecteur de salive et une unité de réception d'échantillon qui se compose d'une unité qui fournit l'air inhalé, comprenant l'embout (1) relié au collecteur de salive (2) au bas et comportant un tuyau (3) à son extrémité, où le tuyau (3) est relié de manière amovible à l'unité de réception d'échantillon au moyen d'un conduit d'entrée (4), dans lequel l'unité de réception d'échantillon se compose d'un tube à essai (6) pour le condensat expiré de l'haleine relié de manière amovible au bas à un bloc de refroidissement (5) équipé d'une chambre de refroidissement (5a) comportant un détecteur de température (5c) et un élément actif du bloc de refroidissement (5b) relié à une unité de dispersion de chaleur (5d) via un système de caloducs, dans lequel le bloc de refroidissement (5) est alimenté par une unité électrique (5f) et contrôlé au moyen d'une unité de contrôle programmable (5e) comportant un affichage LCD et un clavier, dans lequel le bloc de refroidissement (5) est amovible relié à un conduit de sortie (7) auquel l'unité qui force le flux unidirectionnel (8) est reliée, **caractérisé en ce qu'**avant de prélever l'échantillon, le bloc de refroidissement (5) est refroidi à la température T1 préréglée à -10 °C, suite à quoi le bloc de refroidissement (5) est relié par un tuyau (3) à l'unité qui fournit l'air inhalé et par le conduit de sortie (7) à l'unité qui force le flux unidirectionnel (8), suite à quoi l'air expiré depuis les poumons à travers l'embout (1) est dirigé à travers le tuyau (3) vers l'unité de réception d'échantillon dans laquelle l'air fourni par le conduit d'entrée (4) se refroidit dans le bloc de refroidissement (5) à une température inférieure à la température de rosée, à la suite de quoi la vapeur d'eau contenue dans l'air expiré se condense et s'accumule dans le tube à essai (6), tandis que l'unité qui force le flux unidirectionnel (8) est utilisée pour évacuer les gaz, et lorsque une quantité appropriée de condensat expiré est collectée, le tube à essai (6) est déconnecté, dans lequel l'échantillon d'haleine du tube à essai (6) sous forme de condensat de vapeur d'eau est utilisé dans des études ou soumis à un traitement ultérieur visant à fixer l'échantillon, tel que la congélation ou la lyophilisation et soumis à une analyse quantitative et/ou qualitative qui comprend l'analyse du profil d'acides aminés.

2. Procédé selon la revendication 1, dans lequel la cellule à effet Peltier est utilisée comme élément actif du bloc de refroidissement (5b), la puissance fournie par la cellule à effet Peltier (5b) est fixée de sorte qu'elle soit augmentée afin de refroidir le bloc de refroidissement (5), et afin de maintenir la température du bloc de refroidissement (5) au niveau préréglé, elle est réduite, dans lequel la puissance fournie par la cellule à effet Peltier (5b) est contrôlée au moyen de l'unité de contrôle (5e).
